# EUROPEAN PATENT APPLICATION

(11) **EP 4 831 387 A1**
(43) Date of publication of application: **28.10.2026**
(21) Application number: 24907337.0
(22) Date of filing: 13.12.2024
(51) Int. Cl.: D21B 1/06, A61F 13/53, D21H 11/00, D21H 11/04, D21H 11/16

(54) **PULP SHEET FOR FLUFF PULP, FLUFF MAT, ABSORBENT ARTICLE, AND METHOD FOR PRODUCING PULP SHEET FOR FLUFF PULP**

(30) Priority: 22.12.2023 JP 2023216571; 17.05.2024 JP 2024080682
(71) Applicant: Oji Holdings Corporation, Chuo-ku Tokyo 104-0061 (JP)
(72) Inventor: SHIMIZU, Kimiaki, Tokyo 104-0061 (JP); NAKAYAMA, Kodai, Tokyo 104-0061 (JP); ISOZAKI, Tomofumi, Tokyo 104-0061 (JP); WATANABE, Kaori, Tokyo 104-0061 (JP)
(74) Representative: Godemeyer Blum Lenze Patentanwälte Partnerschaft mbB - werkpatent
(86) International application number: PCT/JP2024/044300
(87) International publication number: WO 2025/134952

(57) **Abstract**

It is an object of the present invention to provide a pulp sheet for fluff pulp that suppresses fading and has excellent defibration properties. The present invention relates to a pulp sheet for fluff pulp, in which the pH of a hot water extract thereof, measured in accordance with JIS P 8133-2: 2013, is pH 7.0 to 9.5. Moreover, the present invention relates to a pulp sheet for fluff pulp, comprising a softwood pulp, in which the content of a hardwood pulp with respect to the total pulp components in the pulp sheet for fluff pulp is 30% by mass or less, and the amount of ISO brightness changed is 1.5% or less. Furthermore, the present invention relates to a fluff mat and an absorbent article, which are formed from a pulp sheet for fluff pulp, and a method for producing a pulp sheet for fluff pulp.

## Description

### Technical Field

The present invention relates to a pulp sheet for fluff pulp, a fluff mat, an absorbent article, and a method for producing a pulp sheet for fluff pulp.

### Background Art

Fluff pulp is made by defibrating wood pulp until it becomes a cotton-like state. Such fluff pulp is used as a water-absorbing material in absorbent articles such as disposable diapers and sanitary napkins.

Conventionally, fluff pulp used in absorbent articles is made from softwood pulp with good defibration properties (for example, Patent Document 1). In Patent Document 1, a fluff pulp is obtained by defibrating a raw material pulp (bleached softwood pulp) having a water content percentage of 35% or less through a mechanical treatment.

In addition, Patent Document 2 discloses a pulp sheet for fluff pulp that contains a bleached hardwood pulp and a bleached softwood pulp. In this document, studies are conducted regarding that the content ratio between the bleached hardwood pulp and the bleached softwood pulp is set to be within a predetermined range to obtain a pulp sheet for fluff pulp that can reduce the rewet amount of an absorbed liquid and can suppress skin irritation.

### Prior Art Documents

### Patent Documents

Patent Document1: Japanese Patent Publication No. 2012-211411 A
Patent Document2: International Publication WO2022/138870

### Summary of Invention

### Object to be Solved by the Invention

As mentioned above, a pulp sheet for fluff pulp containing a bleached hardwood pulp and a bleached softwood pulp has been known. However, there have been cases where conventional pulp sheets for fluff pulp sometimes cause color fading under hightemperature conditions, or where the defibration properties of the pulp sheets for fluff pulp were insufficient, thereby requiring improvement.

Thus, in order to solve such problems of the prior art techniques, the present inventors have proceeded with studies for the purpose of providing a pulp sheet for fluff pulp that suppresses color fading and has excellent defibration properties.

### Means for Solving the Object

Examples of specific aspects of the present invention will be shown below.

[1] A pulp sheet for fluff pulp, in which the pH of a hot water extract thereof, measured in accordance with JIS P 8133-2: 2013, is pH 7.0 to 9.5.
[2] The pulp sheet for fluff pulp according to [1], wherein the ISO brightness measured in accordance with JIS P 8148: 2018 is 80% or more.
[3] The pulp sheet for fluff pulp according to [1] or [2], wherein
   the pulp sheet for fluff pulp comprises a softwood pulp, and
   the content of a hardwood pulp with respect to the total pulp components in the pulp sheet for fluff pulp is 30% by mass or less.
[4] The pulp sheet for fluff pulp according to any one of [1] to [3], wherein
   the pulp sheet for fluff pulp comprises a softwood pulp, and
   the content of a hardwood pulp with respect to the total pulp components in the pulp sheet for fluff pulp is 15% by mass or less.
[5] A pulp sheet for fluff pulp, comprising a softwood pulp, in which
   the content of a hardwood pulp with respect to the total pulp components in the pulp sheet for fluff pulp is 30% by mass or less, and
   the amount of ISO brightness changed, calculated according to the following equation, is 1.5% or less: $Amount \left(%\right) of ISO brightness changed = \left(B0 \left(%\right)-B1 \left(%\right)\right) / B 0 \left(%\right) \times 100 ,$
   wherein B1 represents the ISO brightness of the pulp sheet for fluff pulp measured in accordance with JIS P 8154-1: 2008, after being heated at 105°C for 2 hours, and B0 represents the ISO brightness of the pulp sheet for fluff pulp before the heat treatment.
[6] The pulp sheet for fluff pulp according to [5], wherein
   the pulp sheet for fluff pulp comprises a softwood pulp, and
   the content of a hardwood pulp with respect to the total pulp components in the pulp sheet for fluff pulp is 15% by mass or less.
[7] A fluff mat formed from the pulp sheet for fluff pulp according to any one of [1] to [6].
[8] The fluff mat according to [7], wherein after 5 parts by mass of the fluff mat is impregnated with 20 parts by mass of artificial urine containing 1.94% by mass of urea, 0.8040% by mass of sodium chloride, 0.2045% by mass of magnesium sulfate heptahydrate, and 0.0835% by mass of calcium chloride dihydrate, and the resulting fluff mat is then left at rest at 40°C for 1 hour in a sealed state, the pH of a liquid squeezed from the fluff mat is pH 6.6 or more.
[9] An absorbent article, comprising the fluff mat according to [7] or [8].
[10] A method for producing a pulp sheet for fluff pulp, comprising
   cooking a pulp raw material to obtain an unbleached pulp,
   bleaching,
   heating, and forming a sheet formation, wherein
   the heating is provided after the bleaching.
[11] The method for producing a pulp sheet for fluff pulp according to [10], wherein, in the heating, the heat treatment is carried out, so that the pulp slurry temperature becomes 30°C or higher and 100°C or lower.
[12] The method for producing a pulp sheet for fluff pulp according to [10] or [11], wherein the heat treatment time in the heating is 10 minutes or more and 120 minutes or less.
[13] The method for producing a pulp sheet for fluff pulp according to any one of [10] to [12], wherein the concentration of the pulp slurry in the heating is 0.5% by mass or more and 40% by mass or less.
[14] The method for producing a pulp sheet for fluff pulp according to any one of [10] to [13], wherein the pH of the pulp slurry in the heating is pH 2.0 or more and pH 9.0 or less.

### Advantageous Effects of Invention

According to the present invention, a pulp sheet for fluff pulp that suppresses fading and has excellent defibration properties can be obtained.

### Brief Description of Drawings

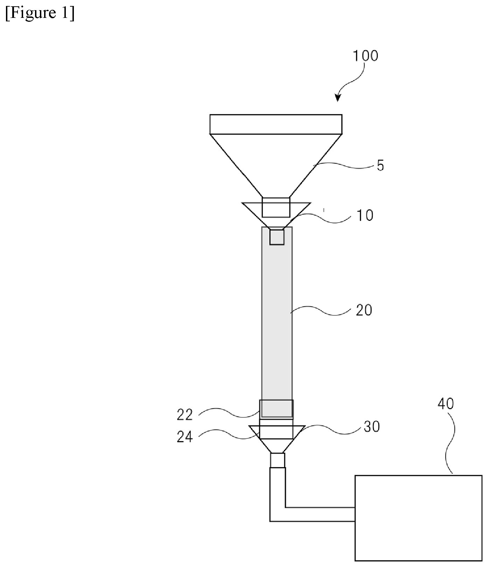

[Figure 1] Figure 1 is a schematic view explaining the configuration of a fluff mat-producing device used in production of fluff mats.

### Embodiments of Carrying out the Invention

The present invention will be described in detail below. The constitutive elements of the present invention given below may be described based on some typical embodiments or specific examples. However, the present invention should not be limited to such embodiments. In the present description, the numerical range expressed with the wording "a number to another number" means the range that falls between the former number indicating the lower limit value of the range and the latter number indicating the upper limit value thereof.

### (Pulp sheet for fluff pulp)

A first embodiment of the present invention relates to a pulp sheet for fluff pulp, in which the pH of a hot water extract thereof, measured in accordance with JIS P 8133-2: 2013, is pH 7.0 to 9.5.

A second embodiment of the present invention relates to a pulp sheet for fluff pulp, comprising a softwood pulp, in which the content of a hardwood pulp with respect to the total pulp components in the pulp sheet for fluff pulp is 30% by mass or less. In addition, in the pulp sheet for fluff pulp of the present embodiment, the amount of ISO brightness changed, which is calculated according to the following equation, is 1.5% or less. $Amount \left(%\right) of ISO brightness changed = \left(B0 \left(%\right)-B1 \left(%\right)\right) / B 0 \left(%\right) \times 100 .$

In the above equation, B1 represents the ISO brightness of the pulp sheet for fluff pulp measured in accordance with JIS P 8154-1: 2008, after being heated at 105°C for 2 hours, and B0 represents the ISO brightness of the pulp sheet for fluff pulp before the heat treatment.

It is to be noted that the "fluff pulp" in the present description means a defibrated pulp, which is a pulp that has been fiberized or has been cut finely. Also, the "pulp sheet for fluff pulp" is a pulp sheet before defibration, which is used to produce a fluff pulp. In other words, the "fluff pulp" is obtained by defibrating the "pulp sheet for fluff pulp," and the "fluff pulp" that has been molded into a sheet is called a "fluff mat." In the present description, the "pulp sheet for fluff pulp" is obtained, for example, by papermaking a slurry containing a raw material pulp.

In the first embodiment, the pulp sheet for fluff pulp is treated with hot water according to the method described in JIS P 8133-2: 2013, and the pH of the obtained hot water extract is pH 7.0 to 9.5. More specifically, 2 g of the pulp sheet for fluff pulp is added to 100 ml of deionized water, and the resulting solution was continuously boiled for 1 hour. Thereafter, the solution was cooled to 25°C, and the contents are filtrated through a glass filter. A 1 mol/L potassium chloride solution is added to the resulting solution to obtain a hot water extract, and the pH thereof is then measured. The pH of the hot water extract is preferably 7.1 or more, more preferably 7.2 or more, and further preferably 7.3 or more. On the other hand, the pH of the hot water extract is preferably 9.4 or less, more preferably 9.3 or less, and further preferably 9.2 or less.

In the first embodiment, for example, in the step of producing a pulp sheet for fluff pulp, after subjecting a pulp raw material to a bleaching treatment, the resultant may be washed, and may be then subjected to a heat treatment under predetermined conditions, so that the pH of the aforementioned hot water extract can be set within a predetermined range. More specifically, it is preferable to set the pH of the slurry during the heat treatment to, for example, pH 2.0 to 9.0. Moreover, even by appropriately adjusting the raw materials for pulp, it becomes easy to set the pH of the aforementioned hot water extract within a predetermined range.

In the first embodiment, the pH of the hot water extract, which is obtained by treating the pulp sheet for fluff pulp with hot water according to the method described in JIS P 8133-2: 2013, is set to be a range of 7.0 to 9.5, so that the fluff pulp can be set to be neutral to alkaline. When a fluff mat formed from such a fluff pulp is used as a constituent material for a diaper, acidic urine can be neutralized, and the occurrence of skin irritation can be suppressed.

For example, 5 parts by mass of a fluff mat is impregnated with 20 parts by mass of artificial urine containing 1.94% by mass of urea, 0.8040% by mass of sodium chloride, 0.2045% by mass of magnesium sulfate heptahydrate, and 0.0835% by mass of calcium chloride dihydrate, and the resulting fluff mat is then left at rest at 40°C for 1 hour in a sealed state. After these operations, the pH of a liquid squeezed from the fluff mat is pH 6.6 or more is preferably 6.6 or more, more preferably 6.8 or more, further preferably 7.0 or more, and particularly preferably 7.5 or more. When the pH of a liquid squeezed from the fluff mat is within the above-described range, it means that the acidic urine has been neutralized, and the occurrence of skin irritation is more effectively suppressed.

Moreover, in the first embodiment, the liquid rewet percentage in the fluff mat is preferably 30% or less, more preferably 28% or less, further preferably 26% or less, particularly preferably 24% or less, and most preferably 20% or less.

The liquid rewet percentage in the fluff mat can be calculated by the formula shown below. Here, the liquid rewet percentage in the fluff mat is calculated when 20 g of artificial urine is absorbed into the fluff mat, and then 10 filter papers (No. 1640, manufactured by ADVANTEC Co., Ltd.) are placed thereon, while pressurizing, under the conditions described in the Examples. The liquid rewet percentage can be calculated by measuring the weight of a liquid absorbed by the filter paper. [Expression 1] $\begin{matrix}\frac{\left(Weight \left[g\right] of 10 filter papers after liquid absorption\right) - \left(Weight \left[g\right] of 10 filter papers before liquid absorption\right)}{20 \left[g\right]} \\ \times 100 \left[%\right]\end{matrix}$

In the second embodiment, the ISO brightness of the pulp sheet for fluff pulp is measured in accordance with JIS P 8148: 2018. At that time, the ISO brightness of the pulp sheet for fluff pulp is measured before and after being heated at 105°C for 120 minutes. After that, the amount of ISO brightness changed is calculated according to the following equation:$Amount of ISO brightness changed = \left(B0\left(%\right)-B1\left(%\right)\right) / B 0 \left(%\right) \times 100 .$

In the above equation, B1 represents the ISO brightness of the pulp sheet for fluff pulp measured in accordance with JIS P 8154-1: 2008, after being heated at 105°C for 2 hours, and B0 represents the ISO brightness of the pulp sheet for fluff pulp before the heat treatment.

The amount of ISO brightness changed is preferably 1.5% or less, more preferably 1.3% or less, and further preferably 1.1% or less. It is to be noted that the lower limit value of the amount of ISO brightness changed is not particularly limited, and that it may also be 0%.

In the second embodiment, for example, in the step of producing a pulp sheet for fluff pulp, by providing a heat treatment step after a pulp raw material has been subjected to a bleaching treatment, the above-described amount of ISO brightness changed can be adjusted to be within a desired range. In particular, it is preferable to perform a hydrogen peroxide treatment in the heat treatment step, so that the amount of ISO brightness changed can be adjusted to be within a desired range. Furthermore, even by appropriately adjusting the raw material for pulp, it becomes easy to set the amount of ISO brightness changed within a predetermined range.

Besides, even in the first embodiment, when the ISO brightness is measured by the same method as the method of measuring the ISO brightness in the second embodiment and when the amount of ISO brightness changed is calculated according to the above equation, the amount of ISO brightness changed is preferably 1.5% or less, more preferably 1.3% or less, and further preferably 1.1% or less. It is to be noted that the lower limit value of the amount of ISO brightness changed is not particularly limited, and that it may also be 0%.

The pulp sheets for fluff pulp of the first embodiment and the second embodiment (hereinafter, the first embodiment and the second embodiment are collectively referred to as "the present embodiment," at times) have favorable defibration properties, when the pulp sheets are fluffed. In the present description, the defibration properties can be evaluated by the defibration time (sec) required for the undefibrated material to disappear. Specifically, the shorter the defibration time, the better the defibration properties that can be determined, and the better the defibration properties, the higher the production efficiency of the fluff mat that can be obtained. Furthermore, the better the defibration properties, the more likely it is that a fluff mat with suppressed liquid rewet can be obtained. Further, the better the defibration properties, the less the amount of an undefibrated material in the fluff mat, so not only does the appearance of the fluff mat is improved, but the ability of the fluff mat to maintain its shape is also improved, and when the fluff mat is used as an absorbent layer for sanitary materials such as disposable diapers, the possibility of the shape of the absorbent layer collapsing due to the movement during use and the resulting deterioration of the absorbent performance of the disposable diapers can be reduced.

In the pulp sheet for fluff pulp of the present embodiment, the ISO brightness measured in accordance with JIS P 8148: 2018 is preferably 80% or more, more preferably 81% or more, further preferably 82% or more, and particularly preferably 83% or more. On the other hand, the ISO brightness of the pulp sheet for fluff pulp is preferably 93% or less. By setting the ISO brightness of the pulp sheet for fluff pulp to be within the above-described range, a pulp sheet for fluff pulp with more excellent appearance can be obtained. Moreover, by setting the ISO brightness of the pulp sheet for fluff pulp to be within the above-described range, a fluff mat with high brightness can be obtained.

The basis weight of the pulp sheet for fluff pulp of the present embodiment is not particularly limited, and the pulp sheet may be produced with a basis weight that enables production by a papermaking device used for papermaking. For example, the basis weight of the pulp sheet for fluff pulp is preferably 100 g/m² or more, more preferably 150 g/m² or more, and further preferably 200 g/m² or more. On the other hand, the basis weight of the pulp sheet for fluff pulp is preferably 1500 g/m² or less, more preferably 1400 g/m² or less, and further preferably 1300 g/m² or less. The basis weight of the pulp sheet for fluff pulp is measured in accordance with JIS P 8124: 2011.

The density of the pulp sheet for fluff pulp of the present embodiment is not particularly limited, and it is preferably 0.3 g/cm³ or more, more preferably 0.4 g/cm³ or more, and further preferably 0.5 g/cm³ or more. On the other hand, the density of the pulp sheet for fluff pulp is preferably 0.95 g/cm³ or less, more preferably 0.90 g/cm³ or less, and further preferably 0.85 g/cm³ or less. The density of the pulp sheet for fluff pulp is measured in accordance with JIS P 8118: 2014.

The burst index of the pulp sheet for fluff pulp of the present embodiment, which is measured in accordance with JIS P 8131: 2009, is preferably 0.5 kPa·m²/g or more, more preferably 0.7 kPa·m²/g or more, and further preferably 0.9 kPa·m²/g or more. On the other hand, the burst index of the pulp sheet for fluff pulp is preferably 5.0 kPa·m²/g or less, more preferably 4.5 kPa·m²/g or less, and further preferably 4.0 kPa·m²/g or less.

### (Raw material pulp)

The pulp sheet for fluff pulp of the present embodiment preferably comprises a softwood pulp. The pulp sheet for fluff pulp of the present embodiment may also comprise a hardwood pulp, but the content of the hardwood pulp with respect to the total pulp components in the pulp sheet for fluff pulp is preferably 30% by mass or less, more preferably 20% by mass or less, further preferably 15% by mass or less, still further preferably 10% by mass or less, and particularly preferably 5% by mass or less. It is to be noted that the content of the hardwood pulp with respect to the total pulp components in the pulp sheet for fluff pulp may also be 0% by mass. By setting the content of the hardwood pulp to be 0% by mass, the defibration properties of the fluff pulp can be effectively enhanced. On the other hand, by allowing the pulp sheet for fluff pulp to contain the hardwood pulp within the above-described range, the rewetting of the liquid absorbed in a fluff mat can be suppressed.

The content of the softwood pulp with respect to the total pulp components in the pulp sheet for fluff pulp softwood pulp is preferably 70% by mass or more, more preferably 80% by mass or more, further preferably 85% by mass or more, still further preferably 90% by mass or more, and particularly preferably 95% by mass or more. Moreover, the upper limit value of the content of a hardwood pulp with respect to the total pulp components in the pulp sheet for fluff pulp is not particularly limited, and it may also be 100% by mass.

Examples of the softwood pulp and the hardwood pulp may include chemical pulps such as a sulfite pulp (SP), a soda pulp (AP), a bleached kraft pulp (BKP), an unbleached kraft pulp (UKP) and an oxygen bleached kraft pulp (OKP), semichemical pulps such as a semi-chemical pulp (SCP) and a chemi-ground wood pulp (CGP), and mechanical pulps such as a ground pulp (GP) and a thermomechanical pulp (TMP, BCTMP). In addition, the softwood pulp or the hardwood pulp may also be a dissolving pulp (DP).

Among others, the softwood pulp comprised in the pulp sheet for fluff pulp is preferably a bleached softwood pulp. The raw materials for the bleached softwood pulp preferably comprise at least one selected from the group consisting of genus Pinus, genus Pseudotsuga and genus Cryptomeria, and more preferably comprise at least one selected from the group consisting of genus Pinus and genus Pseudotsuga. Moreover, in the present embodiment, genus Pinus and genus Pseudotsuga are preferably used in combination.

The Canadian Standard Freeness (CSF) of the softwood pulp measured in accordance with JIS P 8121-2: 2012 is preferably 600 ml or more, more preferably 640 ml or more, and further preferably 670 ml or more. It is to be noted that this Canadian Standard Freeness (CSF) of the softwood pulp is a value measured on a softwood pulp before papermaking.

When a hardwood pulp is comprised in the pulp sheet for fluff pulp, the hardwood pulp is preferably a bleached hardwood pulp. The raw material for the bleached hardwood pulp preferably comprises at least one selected from the group consisting of genus Acacia, genus Eucalyptus, genus Quercus, genus Fagus, and genus Castanopsis, and more preferably comprises at least one selected from the group consisting of genus Acacia and genus Eucalyptus. Moreover, in the present embodiment, as raw materials for the bleached hardwood pulp, both genus Acacia and genus Eucalyptus are preferably used in combination.

The Canadian Standard Freeness (CSF) of the hardwood pulp measured in accordance with JIS P 8121-2: 2012 is preferably 370 ml or more, more preferably 400 ml or more, and further preferably 430 ml or more. It is to be noted that this Canadian Standard Freeness (CSF) of the hardwood pulp is a value measured on a hardwood pulp before papermaking.

The pulp sheet for fluff pulp of the present embodiment may comprise other raw material pulps, in addition to the softwood pulp and the hardwood pulp. Examples of such other raw material pulps may include a non-wood pulp, and a deinked pulp. Examples of the non-wood pulp may include cotton pulps such as cotton linter and cotton lint, bamboo pulps, and non-wood type pulps such as hemp, wheat straw, and bagasse. An example of the deinked pulp may be a deinked pulp using a waste paper as a raw material. In this case, the content of other raw material pulps is preferably 10% by mass or less, with respect to the total pulp components in the pulp sheet for fluff pulp.

The length-weighted average fiber length of the softwood pulp used as a raw material pulp is preferably 1.50 mm or more, more preferably 1.75 mm or more, and further preferably 2.00 mm or more. On the other hand, the length-weighted average fiber length of the softwood pulp is preferably 3.50 mm or less, more preferably 3.20 mm or less, and further preferably 3.00 mm or less.

In addition, when a hardwood pulp is comprised as a raw material pulp, the length-weighted average fiber length of the hardwood pulp is preferably 0.50 mm or more, more preferably 0.60 mm or more, and further preferably 0.65 mm or more. On the other hand, the length-weighted average fiber length of the hardwood pulp is preferably 1.20 mm or less, more preferably 1.10 mm or less, and further preferably 1.0 mm or less.

By setting the fiber lengths of various types of pulps comprised in the pulp sheet for fluff pulp to be within the above-described ranges, the thickness and density of the pulp sheet for fluff pulp can be easily controlled to be in appropriate ranges. Moreover, by setting the fiber lengths of various types of pulps comprised in the pulp sheet for fluff pulp to be within the above-described ranges, when the pulp sheet for fluff pulp is fluffed, the rewetting of the liquid absorbed in the fluff mat can be effectively suppressed. It is to be noted that the length-weighted average fiber lengths of various types of pulps comprised in the pulp sheet for fluff pulp are measured in accordance with JIS P 8226-2: 2011.

### (Optional components)

The pulp sheet for fluff pulp may comprise additives, as necessary. Examples of the additives may include a softener, a filler, a pigment, a sizing agent, a coagulant, an oil-resistant agent, aluminum sulfate, a retention aid, a filtration aid, dry strength agent, wet strength agent, a color pigment, a water-resistant agent, an antistatic agent, sodium sulfate, a surfactant, and a pH adjuster (sodium hydroxide, etc.). The pulp sheet for fluff pulp of the present embodiment may comprise a softener. By allowing the pulp sheet for fluff pulp to comprise a softener, defibration properties can be more effectively increased.

### (Method for producing pulp sheet for fluff pulp)

The method for producing the pulp sheet for fluff pulp of the present embodiment preferably comprises a cooking step of cooking a pulp raw material to obtain an unbleached pulp, a bleaching step, a heat treatment step, and a sheet formation step. It is to be noted that a dewatering and washing step may be appropriately provided between individual steps. In addition, a prehydrolysis step may be provided before the cooking step, an oxygen delignification step may be provided before the bleaching step, and a Cold caustic extraction step may be provided after the bleaching step. Furthermore, a beating step of beating a bleached pulp to a desired freeness may be provided before the sheet formation step.

### (Cooking step)

In the cooking step, a pulp raw material is cooked to obtain an unbleached pulp. A known method can be adopted as a cooking method. The cooking conditions applied in the cooking step are not particularly limited. For example, when a kraft cooking method is used, the degree of sulfidation of a cooking liquid is preferably 15% to 45%, the cooking temperature is preferably 130°C to 170°C, the effective alkali addition percentage is preferably 5% to 30% by mass per oven-dry wood mass, and the cooking time is preferably 45 to 500 minutes. The kappa number of an unbleached pulp obtained through the cooking step is preferably 10 to 70.

### (Prehydrolysis step)

A prehydrolysis step can be provided before the cooking step. In the prehydrolysis step, a hardwood or a softwood is heated in the presence of water to perform a prehydrolysis treatment. Herein, the amount of water relative to the hardwood or the softwood (liquid ratio: the amount of the water (parts by mass)/the amount of the hardwood or the softwood (parts by mass)) is preferably 1.0 to 10, more preferably 1.5 to 8.0, further preferably 2.0 to 7.0, and still further preferably 2.7 to 5.0. When the liquid ratio is 1.0 or more, it is preferable because the hydrolysis proceeds sufficiently and the reaction becomes uniform. When the liquid ratio is 10 or less, it is preferable because the amount of heat required to heat up to a desired temperature is reduced, making it economical. The method of adding water is not particularly limited, and water may be added from the outside, or water originally contained in the hardwood or the softwood may be used, or when the steam is used for heating, water contained in the steam may be utilized. In addition, an alkali, an acid, a chelating agent, etc., which are chemical products that directly or indirectly aid in the hydrolysis of polysaccharides, can be added together with water.

### (Oxygen delignification step)

In the step of producing a pulp sheet for fluff pulp, an oxygen delignification step is preferably provided after the cooking step and before the bleaching step. In the oxygen delignification step, an alkaline chemical and oxygen are added to an unbleached pulp, and the lignin is decomposed at a high temperature and a high pressure, so that it is eluted in the alkaline chemical. The amount of bleaching chemicals used in the subsequent multi-stage bleaching steps can be reduced by delignification in the oxygen delignification step, and a reduction in the pulp quality can be minimized. As an oxygen delignification method, a known medium concentration method or high concentration method can be applied as is, and the medium concentration method, which is currently widely used and is performed at a pulp concentration of 8% to 15% by mass, is preferable.

In the oxygen delignification step performed by the medium concentration method, caustic soda or oxidized kraft white liquor can be used as alkali, and as oxygen gas, oxygen from a cryogenic separation method, oxygen from PSA (Pressure Swing Adsorption), or oxygen from VSA (Vacuum Swing Adsorption) can be used. Such oxygen gas and alkali are added to a medium-concentration pulp slurry in a medium concentration mixer, and after thorough mixing, a mixture of a pulp, oxygen and alkali is sent to a reaction tower capable of retaining the mixture under increased pressure for a certain period of time, and it is then delignified. The oxygen gas addition percentage is preferably 0.5% to 3% by mass per oven-dry pulp mass, the alkali addition percentage is preferably 0.5% to 4% by mass, the reaction temperature is preferably 80°C to 120°C, the reaction time is preferably 15 to 100 minutes, and the pulp concentration is preferably 8% to 15% by mass. As other conditions, known conditions can be applied.

### (Bleaching step)

In the bleaching step, the pulp that has been subjected to oxygen delignification is bleached to obtain a bleached pulp. The bleaching method is not particularly limited, and bleaching can be carried out by a known bleaching method. Examples of the bleaching method may include an ozone bleaching stage (Z), a chlorine dioxide bleaching stage (D), an alkali extraction stage (E), an oxygen bleaching stage (O), a hydrogen peroxide bleaching stage (E/P), a peracid bleaching stage (PA), an acid washing stage (a), and an acid treatment stage (A). In the present embodiment, for example, the bleaching is preferably carried out in the order of D0-E/P-D1. It is to be noted that the Z stage is preferably not performed because it causes great damage to the pulp fibers and the stiffness of the fibers is lost, which deteriorates the defibration properties of the pulp sheet for fluff pulp.

The bleaching conditions in the D0 stage and the D1 stage are not particularly limited, and the chlorine dioxide addition percentage is preferably 0.05% to 5.0% by mass, the bleaching temperature is preferably 30°C to 90°C, the bleaching time is preferably 1 to 240 minutes, the pulp concentration is preferably 5% to 15% by mass, and pH at the time of termination of the reaction is preferably pH 2 to 6. In addition, the bleaching conditions in the E/P stage are not particularly limited, and the addition percentage of hydrogen peroxide is preferably 0.05% to 5.0% by mass, the bleaching temperature is preferably 30°C to 90°C, the bleaching time is preferably 1 to 240 minutes, the pulp concentration is preferably 5% to 15% by mass, and the pH at the time of termination of the reaction is preferably pH 10 to 12.

### (Cold caustic extraction step)

In the present embodiment, the pulp that has been bleached is further subjected to an alkali treatment, so that a bleached pulp after the cold alkali treatment may be obtained. The treatment method is not particularly limited, and caustic soda can be used as alkali. The caustic soda addition percentage is preferably 5.0% to 20.0%, the bleaching temperature is preferably 20°C to 50°C, the bleaching time is preferably 1 to 240 minutes, and the pulp concentration is preferably 5% to 15% by mass.

### (Heat treatment step)

In the present embodiment, it is preferable to provide a heat treatment step after the bleaching step, the Cold caustic extraction step, or the washing step. In the heat treatment step, the pulp slurry is retained at a temperature higher than room temperature for 30 minutes or more. The temperature of the pulp slurry in the heat treatment step is preferably 30°C or higher, more preferably 35°C or higher, and further preferably 40°C or higher. On the other hand, the temperature of the pulp slurry in the heat treatment step is preferably 100°C or lower, more preferably 90°C or lower, further preferably 80°C or lower, and particularly preferably 60°C or lower. It is to be noted that while the temperature of the pulp slurry in the heat treatment step is preferable within the above-described range, it does not necessarily have to be a constant temperature.

The heating time in the heat treatment step may be 1 minute or more, 10 minutes or more, 20 minutes or more, 30 minutes or more, 1 hour or more, 2 hours or more, 3 hours or more, 5 hours or more, 8 hours or more, or 10 hours or more. On the other hand, the heating time in the heat treatment step is preferably 100 hours or less.

The pH of the pulp slurry in the heat treatment step is preferably 2.0 or more, more preferably 2.5 or more, even more preferably 3.0 or more, further preferably 4.0 or more, and particularly preferably 5.0 or more. On the other hand, the pH of the pulp slurry in the heat treatment step is preferably 9.0 or less, more preferably 8.5 or less, and further preferably 8.0 or less. It is to be noted that while the pH of the pulp slurry in the heat treatment step is preferable within the above-described range, it does not necessarily have to be a constant pH.

The concentration of the pulp slurry in the heat treatment step is preferably 0.5% by mass or more, more preferably 2% by mass or more, and further preferably 5% by mass or more. On the other hand, the concentration of the pulp slurry is preferably 40% by mass or less, more preferably 35% by mass or less, and further preferably 30% by mass or less. It is to be noted that while the concentration of the pulp slurry in the heat treatment step is preferable within the above-described range, it does not necessarily have to be a constant concentration.

In the present embodiment, by setting the heating conditions and the pH of the pulp slurry in the heat treatment step to be within the above-described range, it becomes easy to set the pH of the hot water extract obtained by treating the pulp sheet for fluff pulp with hot water using the method described in JIS P 8133-2:2013 to be within the range of pH 7.0 to 9.5.

The heating method in the heat treatment step is not particularly limited. Steam may be directly introduced into the pulp slurry for heating, a jacketed heating device may be used, or the slurry may be left at rest in a heated atmosphere at a temperature higher than room temperature. Alternatively, it may also be a method of using the temperature of the pulp slurry that is maintained from the previous step for heating. When a heating container is used in the heat treatment step, it may be made of concrete, metal, or resin, as material. As such a heating container, a dedicated heating tank may be used , or the retention time up to the sheet formation step may be utilized.

Besides, the present embodiment comprises a cooking step of cooking a pulp raw material to obtain an unbleached pulp, a bleaching step, a washing step, a heat treatment step, and a sheet formation step. The pH of the pulp slurry in the heat treatment step is preferably 2.0 or more, more preferably 2.5 or more, even more preferably 3.0 or more, further preferably 4.0 or more, and particularly preferably 5.0 or more. On the other hand, the pH of the pulp slurry in the heat treatment step is preferably 9.0 or less, more preferably 8.5 or less, and further preferably 8.0 or less. It is to be noted that while the pH of the pulp slurry in the heat treatment step is preferable within the above-described range, it does not necessarily have to be a constant pH.

In the present embodiment, the above-mentioned heat treatment step may be a color tone adjustment step. When color tone adjustment is performed in the heat treatment step, a hydrogen peroxide treatment is preferable. It is to be noted that, in the final step of the bleaching step, a hydrogen peroxide treatment step is provided, this step corresponds to the color tone adjustment step. In addition, a hydrogen peroxide treatment step may also be provided separately from the bleaching step.

When a hydrogen peroxide treatment step is provided in the heat treatment step, the hydrogen peroxide treatment time is preferably 1 minute or more, more preferably 10 minutes or more, further preferably 20 minutes or more, and particularly preferably 30 minutes or more. On the other hand, the hydrogen peroxide treatment time is preferably 600 minutes or less, more preferably 300 minutes or less, and further preferably 120 minutes or less.

When a hydrogen peroxide treatment step is provided in the heat treatment step, the concentration of the pulp slurry is preferably 0.5% by mass or more, more preferably 2% by mass or more, and further preferably 5% by mass or more. On the other hand, the concentration of the pulp slurry is preferably 40% by mass or less, more preferably 35% by mass or less, and further preferably 30% by mass or less. It is to be noted that while the concentration of the pulp slurry in the hydrogen peroxide treatment step is preferable within the above-described range, it does not necessarily have to be a constant concentration.

In the present embodiment, by providing a hydrogen peroxide treatment step, it becomes easy to adjust the amount of ISO brightness changed within a desired range.

### (Washing step)

A washing step may be provided after the aforementioned steps. The washing step is preferably a dewatering and washing step. In the dewatering and washing step, the pulp obtained after each step is diluted, and is washed by combining it with dehydration. The washing method is not particularly limited, and the washing can be performed using a known washing machine. For example, a replacement washing method, a dilution/dehydration method, a press washing method, or a combination of these washing methods, can be used.

It is to be noted that the washing step is preferably provided after the bleaching step, but that the washing step is not limited to after the bleaching step, and it may be provided between individual steps, as necessary.

### (Sheet formation step)

In the sheet formation step, first, the bleached pulp obtained through the heat treatment step is dispersed in water to prepare a slurry. The pulp concentration in the slurry is preferably 0.01% to 10% by mass. In addition, if additives are added as necessary, it is preferable to add the additives into the slurry.

The sheet formation step includes a step of making a paper from the slurry using a papermaking machine. The papermaking machine used in the papermaking step is not particularly limited, and for example, papermaking machines such as a fourdrinier papermaking machine, a cylinder papermaking machine, a hybrid former and a gap former, can be used. In addition, when making a paper, the paper may be made in a single layer, or multiple layers may be combined together to make a paper.

### (Fluff mat)

The present embodiment relates to a fluff mat formed from the aforementioned pulp sheet for fluff pulp. The fluff mat in the present description is obtained by defibrating the aforementioned pulp sheet for fluff pulp to obtain a fluff pulp, and then molding the obtained fluff pulp into a sheet shape. If the fluff mat of the present embodiment is used, for example, as a constituent material for a diaper, it can suppress the occurrence of skin irritation. Moreover, the fluff mat of the present embodiment has excellent water absorption properties, and can retain a liquid after it has absorbed the liquid. For this reason, the rewetting of the liquid can be suppressed in the fluff mat of the present embodiment.

The conditions for defibrating the pulp sheet for fluff pulp are not particularly limited, and for example, a known defibrating machine or grinding machine can be used.

The fluff mat is formed by stacking the fluff pulp under an air flow. Specifically, the defibrated fluff pulp is transported by air and is then stacked on a metal net or on a porous substrate such as a tissue or a nonwoven fabric.

### (Absorbent article)

The present embodiment may relate to an absorbent article having the aforementioned fluff mat. Examples of the absorbent article may include disposable diapers, incontinence pads, light incontinence pads, and sanitary napkins. Since the fluff mat of the present embodiment can neutralize, in particular, acidic urine, the present absorbent article is preferably a disposable diaper, an incontinence pad, or a light incontinence pad.

### Examples

Hereinafter, the features of the present invention will be more specifically described with reference to Examples and Comparative Examples. The materials, used amounts, proportions, treatment content, treatment procedures, and the like shown in the following Examples can be appropriately changed to the extent that such changes do not depart from the spirit of the present invention. Therefore, the scope of the present invention should not be construed as being limited by the following specific examples.

### (First embodiment)

### [Preparation of softwood pulp]

### [Cooking]

After the chips of genus Pinus had been mixed with the chips of genus Pseudotsuga at an oven-dry mass ratio of 30 : 70, 200 g (oven-dry mass) of the mixed chips were collected and placed in a 2.5-L autoclave, and kraft cooking was then carried out under the conditions of: a liquid ratio of 5, a degree of sulfidation of a cooking liquid of 28%, an alkali addition percentage of 22%, a cooking temperature of 165°C, and a cooking time of 120 minutes. Thereafter, a black liquor and a pulp were separated from each other, and the pulp was refined using a flat screen equipped with a 10-cut screen plate to obtain an unbleached pulp with a kappa number of about 30.

### [Oxygen delignification]

The unbleached pulp (70.0 g) was collected at an oven-dry mass, and caustic soda was then added to the unbleached pulp, so that the amount of the caustic soda became 2.0% by mass with respect to the mass of the oven-dried pulp. Subsequently, the obtained mixture was diluted with ion exchange water to adjust the pulp concentration to 10% by mass. This reaction mixture was placed in a heat-type autoclave, and 99.9% commercially available compressed oxygen gas was then injected into the autoclave to set the gauge pressure at 0.5 MPa. Thereafter, oxygen delignification was carried out at 95°C for 60 minutes. The obtained pulp was diluted with ion exchange water to 3% by mass, and was then dehydrated and washed in a Buchner funnel.

### [Bleaching]

The kraft pulp (60 g) after alkaline oxygen delignification was collected at an oven-dry mass, and was then placed in a plastic bag. The pulp concentration was adjusted to 10% by mass using ion exchange water, and chlorine dioxide was added to the pulp so that the amount of chlorine dioxide became 2.0% by mass with respect to the mass of the oven-dried pulp. This reaction mixture was immersed in a thermostatic water bath at 70°C for 60 minutes to carry out a D0 stage treatment. Thereafter, the obtained pulp was diluted with ion exchange water to 3% by mass, and was then dehydrated and washed in a Buchner funnel.

The pulp after the D0 stage was placed in a plastic bag, and the pulp concentration was adjusted to 10% by mass by addition of ion exchange water. Thereafter, caustic soda and hydrogen peroxide were added to the pulp, so that the amount of the caustic soda became 1.0% by mass and the amount of the hydrogen peroxide became 0.2% by mass with respect to the mass of the oven-dried pulp, followed by full mixing. Thereafter, the reaction mixture was immersed in a thermostatic water bath at 70°C for 90 minutes to carry out an E/P stage treatment. The obtained pulp was diluted with ion exchange water to 3% by mass, and was then dehydrated and washed in a Buchner funnel.

The pulp after the E/P stage was placed in a plastic bag, and the pulp concentration was adjusted to 10% by mass by addition of ion exchange water. Thereafter, caustic soda and hydrogen peroxide were added to the pulp, so that the amount of the caustic soda became 1.0% by mass and the amount of the hydrogen peroxide became 0.3% by mass with respect to the mass of the oven-dried pulp, followed by fully mixing. Thereafter, the reaction mixture was immersed in a thermostatic water bath at 70°C for 90 minutes to carry out a P stage treatment. The obtained pulp was diluted with ion exchange water to 3% by mass, and was then dehydrated and washed in a Buchner funnel.

The pulp after the P stage was placed in a plastic bag, and the pulp concentration was adjusted to 10% by mass by addition of ion exchange water. Thereafter, chlorine dioxide was added to the pulp, so that the amount of the chlorine dioxide became 0.2% by mass with respect to the mass of the oven-dried pulp, and the reaction mixture was then immersed in a thermostatic water bath at 70°C for 60 minutes to carry out a D1 stage bleaching. The obtained bleached softwood pulp was diluted with ion exchange water to 3% by mass, and was then dehydrated and washed in a Buchner funnel.

The pulp after the D1 stage was placed in a plastic bag, and the pulp concentration was adjusted to 10% by mass by addition of ion exchange water. Thereafter, chlorine dioxide was added to the pulp, so that the amount of the chlorine dioxide became 1.0% by mass with respect to the mass of the oven-dried pulp, and the reaction mixture was then immersed in a thermostatic water bath at 70°C for 60 minutes to carry out a D2 stage bleaching. The obtained bleached softwood pulp was diluted with ion exchange water to 3% by mass, and was then dehydrated and washed in a Buchner funnel, to obtain a bleached softwood pulp slurry having a concentration of 10% by mass.

### [Preparation of hardwood pulp]

### [Cooking]

After the chips of genus Eucalyptus had been mixed with the chips of genus Acacia at an oven-dry mass ratio of 30 : 70, 200 g (oven-dry mass) of the mixed chips were collected and placed in a 2.5-L autoclave, and kraft cooking was then carried out under the conditions of: a liquid ratio of 5, a degree of sulfidation of a cooking liquid of 28%, an alkali addition percentage of 20%, a cooking temperature of 155°C, and a cooking time of 120 minutes. Thereafter, a black liquor and a pulp were separated from each other, and the pulp was refined using a flat screen equipped with an 8-cut screen plate to obtain an unbleached pulp with a kappa number of about 18.

### [Oxygen delignification]

The unbleached pulp (70.0 g) was collected at an oven-dry mass, and caustic soda was then added to the unbleached pulp, so that the amount of the caustic soda became 2.0% by mass with respect to the mass of the oven-dried pulp. Subsequently, the obtained mixture was diluted with ion exchange water to adjust the pulp concentration to 10% by mass. This reaction mixture was placed in a heat-type autoclave, and 99.9% commercially available compressed oxygen gas was then injected into the autoclave to set the gauge pressure at 0.5 MPa. Thereafter, oxygen delignification was carried out at 95°C for 60 minutes. The obtained pulp was diluted with ion exchange water to 3% by mass, and was then dehydrated and washed in a Buchner funnel.

### [Bleaching]

The kraft pulp (60 g) after the alkaline oxygen delignification was collected at an oven-dry mass, and was placed in a plastic bag. The pulp concentration was adjusted to 10% by mass with ion exchange water, and chlorine dioxide was then added thereto so that the amount of the chlorine dioxide became 1.0% by mass with respect to the mass of the oven-dried pulp. This reaction mixture was immersed in a thermostatic water bath at 70°C for 60 minutes to perform a D0 stage treatment. The obtained pulp was diluted with ion exchange water to 3% by mass, and was then dehydrated and washed in a Buchner funnel.

The pulp after the D0 stage was placed in a plastic bag, and the pulp concentration was adjusted to 10% by mass by addition of ion exchange water. Thereafter, caustic soda and hydrogen peroxide were added to the pulp, so that the amount of the caustic soda became 1.0% by mass and the amount of the hydrogen peroxide became 0.2% by mass with respect to the mass of the oven-dried pulp, followed by full mixing. Thereafter, the reaction mixture was immersed in a thermostatic water bath at 70°C for 90 minutes to carry out an E/P stage treatment. The obtained pulp was diluted with ion exchange water to 3% by mass, and was then dehydrated and washed in a Buchner funnel.

The pulp after the E/P stage was placed in a plastic bag, and the pulp concentration was adjusted to 10% by mass by addition of ion exchange water. Thereafter, chlorine dioxide was added to the pulp, so that the amount of the chlorine dioxide became 0.2% by mass with respect to the mass of the oven-dried pulp, and the reaction mixture was then immersed in a thermostatic water bath at 70°C for 60 minutes to carry out a D1 stage bleaching. The obtained bleached hardwood pulp was diluted with ion exchange water to 3% by mass, and was then dehydrated and washed in a Buchner funnel.

The pulp after the D1 stage was placed in a plastic bag, and the pulp concentration was adjusted to 10% by mass by addition of ion exchange water. Thereafter, chlorine dioxide was added to the pulp, so that the amount of the chlorine dioxide became 1.0% by mass with respect to the mass of the oven-dried pulp, and the reaction mixture was then immersed in a thermostatic water bath at 70°C for 60 minutes to carry out a D2 stage bleaching. The obtained bleached hardwood pulp was diluted with ion exchange water to 3% by mass, and was then dehydrated and washed in a Buchner funnel, to obtain a bleached hardwood pulp slurry having a concentration of 10% by mass.

### (Example 1)

The bleached softwood pulp slurry (60 g) having a concentration of 10% by mass after the D2 stage was placed in a plastic bag, and sulfuric acid was then added thereto to adjust the pH of the pulp slurry to pH 3.0. The resulting pulp slurry was left at rest at 40°C for 12 hours, and a heat treatment was carried out. Thereafter, the treated pulp was diluted to a concentration of about 1.0% by mass by addition of ion exchange water, and then, using a standard circular hand papermaking machine (manufactured by KUMAGAI RIKI KOGYO Co., Ltd.), the reaction mixture was subjected to papermaking, so that the basis weight became 750 g/m², thereby obtaining a pulp sheet for fluff pulp.

### (Example 2)

A pulp sheet for fluff pulp was obtained by performing the same operations as those in Example 1, with exception that, in Example 1, caustic soda was added to the bleached softwood pulp slurry having a concentration of 10% by mass to adjust the pH of the pulp slurry to pH 5.5.

### (Example 3)

A pulp sheet for fluff pulp was obtained by performing the same operations as those in Example 1, with exception that, in Example 1, caustic soda was added to the bleached softwood pulp slurry having a concentration of 10% by mass to adjust the pH of the pulp slurry to pH 8.0.

### (Example 4)

A pulp sheet for fluff pulp was obtained by performing the same operations as those in Example 1, with exception that, in Example 2, ion exchange water was added to the bleached softwood pulp slurry, so that the concentration of the bleached softwood pulp slurry during the heat treatment was adjusted to 2% by mass.

### (Example 5)

A pulp sheet for fluff pulp was obtained by performing the same operations as those in Example 1, with exception that, in Example 2, the bleached softwood pulp slurry was dehydrated, so that the concentration of the bleached softwood pulp slurry was adjusted to 35% by mass.

### (Example 6)

A pulp sheet for fluff pulp was obtained by performing the same operations as those in Example 1, with exception that, in Example 2, the heat treatment temperature for the bleached softwood pulp slurry was set to be 70°C.

### (Example 7)

A pulp sheet for fluff pulp was obtained by performing the same operations as those in Example 1, with exceptions that, in Example 2, the heat treatment temperature for the bleached softwood pulp slurry was set to be 60°C, and the bleached softwood pulp slurry was then allowed to cool so that the temperature became 25°C after 12 hours.

### (Example 8)

A pulp sheet for fluff pulp was obtained by performing the same operations as those in Example 1, with exception that, in Example 2, the heat treatment time for the bleached softwood pulp slurry was set to be 2 hours.

### (Example 9)

A pulp sheet for fluff pulp was obtained by performing the same operations as those in Example 1, with exception that, in Example 2, the heat treatment time for the bleached softwood pulp slurry was set to be 48 hours.

### (Example 10)

A pulp sheet for fluff pulp was obtained by performing the same operations as those in Example 2, with exception that, in "Preparation of softwood pulp," the chips of genus Pinus were mixed with the chips of genus Pseudotsuga at an oven-dry mass ratio of 70 : 30

### (Example 11)

The heat treatment was carried out on the bleached softwood pulp slurry having a concentration of 10% by mass in the same manner as that of Example 2. On the other hand, the bleached hardwood pulp slurry (60 g) having a concentration of 10% by mass after the D2 stage was placed in a plastic bag, and caustic soda was then added thereto to adjust the pH of the pulp slurry to pH 5.5. The resulting pulp slurry was left at rest at 40°C for 12 hours, and a heat treatment was carried out on the bleached hardwood pulp. Thereafter, the bleached softwood pulp and the bleached hardwood pulp, which had been subjected to the heat treatment, were mixed with each other, so that the oven-dry mass ratio became 95 : 5 during papermaking. Except for these operations, the same operations as those in Example 1 were carried out to obtain a pulp sheet for fluff pulp.

### (Example 12)

A pulp sheet for fluff pulp was obtained by performing the same operations as those in Example 11, with exception that, in Example 11, the bleached softwood pulp was mixed with the bleached hardwood pulp at an oven-dry mass ratio of 85 : 15 during papermaking.

### (Example 13)

A pulp sheet for fluff pulp was obtained by performing the same operations as those in Example 11, with exception that, in Example 11, the bleached softwood pulp was mixed with the bleached hardwood pulp at an oven-dry mass ratio of 70 : 30 during papermaking.

### (Example 14)

A pulp after the D1 stage was prepared in "Bleaching" in each of "Preparation of softwood pulp" and "Preparation of hardwood pulp." The bleached softwood pulp after the D1 stage and the bleached hardwood pulp after the D1 stage were mixed with each other, so that the oven-dry mass ratio became 85 : 15, thereby obtaining a mixed pulp slurry having a concentration of 10% by mass, comprising the bleached softwood pulp and the bleached hardwood pulp. Thereafter, caustic soda was added to the mixed pulp slurry having a concentration of 10% by mass, so that the pH of the pulp slurry was set to be pH 5.5. Except for these operations, the same operations as those in Example 2 were carried out.

### (Comparative Example 1)

A pulp sheet for fluff pulp was obtained by performing the same operations as those in Example 1, with exception that, in Example 1, the heat treatment was not carried out.

### (Comparative Example 2)

A pulp sheet for fluff pulp was obtained by performing the same operations as those in Example 1, with exception that, in Example 1, the pH of the bleached softwood pulp was set to be pH 1.0.

### (Comparative Example 3)

A pulp sheet for fluff pulp was obtained by performing the same operations as those in Example 1, with exception that, in Example 1, the pH of the bleached softwood pulp was set to be pH 10.

### (Comparative Example 4)

A pulp sheet for fluff pulp was obtained by performing the same operations as those in Example 11, with exception that, in Example 11, the bleached softwood pulp was mixed with the bleached hardwood pulp at an oven-dry mass ratio of 50 : 50 during papermaking.

### (Comparative Example 5)

The same operations as those in Example 1 were carried out, with the exception that, in Example 2, the bleached softwood pulp slurry having a concentration of 10% by mass after the D2 stage was changed to the bleached hardwood pulp slurry having a concentration of 10% by mass after the D2 stage.

### (Measurement/Evaluation)

### [ISO brightness]

The ISO brightness of each pulp was measured in accordance with JIS P 8148: 2018.

### [pH of extract]

The pH values of the hot water extracts of the pulp sheets for fluff pulp obtained in the examples and the comparative examples were measured in accordance with JIS P 8133-2: 2013.

### [Color fading (amount of brightness changed)]

Five pulp sheets for fluff pulp obtained in each of the examples and the comparative examples were placed in an air dryer set to 105°C. One sheet was removed at 0 minutes, 15 minutes, 30 minutes, 60 minutes, and 120 minutes later. After humidity had been conditioned under an environment of 23°C and a relative humidity of 50% for 4 hours, the ISO brightness was measured in accordance with JIS P 8148:2018. Color fading (amount of brightness changed) was calculated using the following equation. 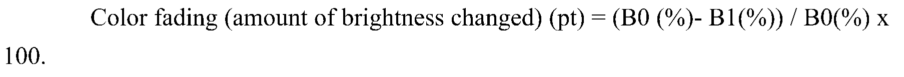Color fading (amount of brightness changed) (pt) = (B0 (%)- B1(%)) / B0(%) x 100.

Herein, B0 represents the ISO brightness of the pulp sheet for fluff pulp 0 minutes later, and B1 represents the ISO brightness of the pulp sheet for fluff pulp 120 minutes later.

### [Defibration properties]

The pulp sheet for fluff pulp, which had been humidity-conditioned under an environment of 23°C and a relative humidity of 50%, was cut into a 10 mm x 10 mm square, and 3 g thereof was weighed out and was put into a Wonder Blender (manufactured by OSAKA CHEMICAL Co., Ltd., model: WB-1, rotation number: 25000 rpm) while avoiding any bias. The pulp was defibrated for a predetermined time to prepare a fluff pulp. Subsequently, the fluff pulp was subjected to a standard circular hand papermaking machine to prepare a handmade paper with a basis weight of 25 g/m². The time required for undefibrated materials to disappear by visual observation is defined as a defibration time.

### [Production of fluff mat]

The fluff mat was produced under an environment of 23°C and a relative humidity of 50%, using a fluff mat-producing device 100 shown in Figure 1. Herein, the fluff mat-producing device 100 having the configuration as shown in Figure 1 was assembled, using a pulp defibrating machine 5 (manufactured by Maruto Seiki), a stainless steel sieve 22 with a sieve opening of 180 µm (manufactured by AS ONE CORPORATION, inner diameter 75 mm x inner height 20 mm), a stainless steel sieve 24 with a sieve opening of 150 µm (manufactured by AS ONE CORPORATION, inner diameter 75 mm x inner height 20 mm), a column 20 prepared using a PET resin plate (manufactured by Acrysunday Co., Ltd., Sunday PET, thickness 0.5 mm) cut into 70 cm x 40 cm, a polypropylene powder funnel 10 (manufactured by AS ONE CORPORATION, model No. 166, aperture: 180 mm, foot outer diameter: 43 mm, foot length: 48 mm), a polypropylene powder funnel 30 (manufactured by AS ONE CORPORATION, model No. 169, aperture: 120 mm, foot outer diameter: 30 mm, foot length: 27 mm), and a household vacuum cleaner 40 (manufactured by Hitachi Home & Life Solutions, Inc., model: CV-CF4). First, the total weight of the stainless steel sieve with a sieve opening of 180 µm and the column was measured in advance. Next, the pulp defibrating machine and the household vacuum cleaner were started up. The suction power of the household vacuum cleaner was set to be low (suction power of approximately 80 W), and the fluff pulp without undefibrated materials obtained during the evaluation in [Defibration properties] was added little by little from the top of the fluff mat-producing device. The weight of the fluff mat formed on the stainless steel sieve with a sieve opening of 180 µm was measured from time to time, and the fluff mat was formed until the weight reached 200 g/m². After the weight had reached 200 g/m², the pulp defibrating machine and the household vacuum cleaner were stopped. After removing the column, the surface of the fluff mat was lightly pressed with a circular stainless steel plate to shape it, and the fluff mat (diameter: 75 mm) was gently removed from the sieve with a sieve opening of 180 µm without losing its shape. The surface that had been in contact with the stainless steel mesh was defined to be the wire surface of the fluff mat.

### [Measurement of rewet percentage of fluff mat]

The rewet percentage of the fluff mat was measured under an environment of 23°C and a relative humidity of 50%. The produced 200 g/m² fluff mat was placed on a 5 mm thick acrylic plate with the wire side facing up. After a 2 mm thick spacer had been inserted therebetween, another 5 mm thick acrylic plate was further placed on the top, and was pressed with a tabletop pressing machine for 10 minutes. After completion of the pressing, the acrylic plate placed on the top was removed, and the resultant was then left at rest for 30 minutes. A stainless steel base plate (5 mm thick, 11 x 11 cm) was placed on a horizontal laboratory table, and one fluff mat was then placed thereon with the wire side facing up. Thereafter, SAP (SA60SXII, manufactured by Sumitomo Seika Chemicals Co., Ltd.) was sprinkled evenly so that SAP/fluff mat = 0.35, and another one fluff mat was placed thereon with the wire side facing up (the total basis weight of the fluff mats: 400 g/m²). An 11 cm x 11 cm square core wrap tissue (Oji Nepia Co., Ltd., basis weight: 16 g/m²) was placed on the top of the fluff mats, so that the paper flow direction became a longitudinal direction. On the top thereof, a stainless steel-made strike-through plate (10 cm x 10 cm, thickness: 85 mm, weight: 665 g) with a 25 mm diameter hole in the center thereof was placed, and after 10 seconds, 20 g of artificial urine was poured into the hole of the strike-through plate. Immediately after the artificial urine had been completely absorbed, the strike-through plate was removed. Next, an 11 cm x 11 cm square polyethylene sheet was placed on the top of the core wrap tissue, and an 11 cm x 11 cm square sponge rubber sheet made of chloroprene rubber (manufactured by AS ONE CORPORATION, thickness: 10 mm, hardness (Asker C hardness scale): 45 degrees) was placed on the top of the polyethylene sheet, and a 2.2 kg weight was placed on the top thereof. Since the total weight of the polyethylene sheet, the sponge rubber sheet, the two acrylic plates, and the 2.2 kg weight was 2.31 kg, and the area of the fluff mat was 0.00442 m², the pressure applied to the fluff mat was 5.1 kPa. The mat was pressurized at a pressure of 5.1 kPa for 3 minutes. Subsequently, the polyethylene sheet, the sponge rubber sheet, the two acrylic plates, and the 2.2 kg weight were removed, and 10 filter papers (11 cm x 11 cm square, manufactured by ADVANTEC, No. 1640) that had been humidity-conditioned under an environment of 23°C and a relative humidity of 50% and had a known weight were placed on the mat, so that the front side of the filter paper was allowed to come into contact with the core wrap tissue for disposable diapers. Then, a clear file, a urethane rubber sheet, two acrylic plates, and a 2.2 kg weight were placed on the mat again, and the filter papers were allowed to absorb the artificial urine while applying pressure for 2 minutes. After 2 minutes, the 10 filter papers were removed and weighed, and the rewet percentage was calculated using the formula described below. The artificial urine used herein was prepared by the following procedures. First, methylene blue (manufactured by Kanto Chemical Co., Inc.) was dissolved in ion exchange water to prepare a and diluted to a methylene blue aqueous solution with a concentration of 0.50% by mass. Next, 19.40 g of urea (manufactured by Kanto Chemical Co., Inc.), 8.040 g of sodium chloride (manufactured by Kanto Chemical Co., Inc.), 2.045 g of magnesium sulfate heptahydrate (manufactured by Kanto Chemical Co., Inc.), and 0.835 g of calcium chloride dihydrate were successively weighed out in a 500 ml beaker, and after adding an appropriate amount of ion exchange water, 1.50 g of 0.50 mass % methylene blue solution was added dropwise thereto. Subsequently, the resulting solution was transferred into a 1 L measuring flask and made up to 1 L to prepare artificial urine. [Expression 2] $\begin{matrix}\frac{\left(Weight \left[g\right] of 10 filter papers after liquid absorption\right) - \left(Weight \left[g\right] of 10 filter papers before liquid absorption\right)}{20 \left[g\right]} \\ \times 100 \left[%\right]\end{matrix}$

### [pH obtained when fluff pulp is mixed with artificial urine]

The pulp sheet for fluff pulp, which had been humidity-conditioned under an environment of 23°C and a relative humidity of 50%, was cut into a 10 mm x 10 mm square, and 3 g thereof was weighed out and was put into a Wonder Blender (manufactured by OSAKA CHEMICAL Co., Ltd., model: WB-1, rotation number: 25000 rpm) while avoiding any bias, and it was then defibrated for 20 seconds to produce a fluff pulp. The fluff pulp (5 g) produced by the above-described method was weighed out, and was then spread thinly on aluminum foil. The artificial urine (20 g), used in "Measurement of rewet percentage of fluff mat," was sprinkled over it using a pipette. Thereafter, the fluff pulp was then placed in a resealable plastic bag and was then left at rest in a 40°C oven for 1 hour. After 1 hour, the fluff pulp was squeezed by hand, and the pH of the squeezed liquid was measured.

### [Sensory evaluation (skin irritation)]

Disposable diapers were produced using fluff mats formed from the pulp sheets for fluff pulp of the examples and the comparative examples, with reference to the description in Japanese Patent No. 5849759. Twenty monitors wore these diapers, and the presence or absence of skin irritation was evaluated. The evaluation criteria were as follows:
A: 0 person experienced skin irritation
B: 1 or 2 persons experienced skin irritation
C: 3 or 4 persons experienced skin irritation
D: 5 or more persons experienced skin irritation

**[Table 1]**

| | | | | | Example 1 | Example 2 | Example 3 | Example 4 | Example 5 | Example 6 | Example 7 | Example 8 | Example 9 | Example 10 | Example 11 | Example 12 | Example 13 | Example 14 |
|---|---|---|---|---|---|---|---|---|---|---|---|---|---|---|---|---|---|---|
| Hardwood pulp | Tree type | genus Acacia | | % | - | - | - | - | - | - | - | - | - | - | 70 | 70 | 70 | 70 |
| | | genus Eucalyptus | | % | - | - | - | - | - | - | - | - | - | - | 30 | 30 | 30 | 30 |
| | Heat treatment | Cond. | Time | h | - | - | - | - | - | - | - | - | - | - | 12 | 12 | 12 | 12 |
| | | | Temperature | °C | - | - | - | - | - | - | - | - | - | - | 40 | 40 | 40 | 40 |
| | | | Pulp conc. | % | - | - | - | - | - | - | - | - | - | - | 10 | 10 | 10 | 10 |
| | | | pH | - | - | - | - | - | - | - | - | - | - | - | 5.5 | 5.5 | 5.5 | 5.5 |
| | | Brightness | | % | - | - | - | - | - | - | - | - | - | - | 87.4 | 87.4 | 87.4 | - |
| Softwood pulp | Tree type | genus Pseudotsuga | | % | 70 | 70 | 70 | 70 | 70 | 70 | 70 | 70 | 70 | 30 | 70 | 70 | 70 | 70 |
| | | genus Pinus | | % | 30 | 30 | 30 | 30 | 30 | 30 | 30 | 30 | 30 | 70 | 30 | 30 | 30 | 30 |
| | Heat treatment | Cond. | Time | h | 12 | 12 | 12 | 12 | 12 | 12 | 12 | 4 | 48 | 12 | 12 | 12 | 12 | 12 |
| | | | Temperature | °C | 40 | 40 | 40 | 40 | 40 | 70 | 60→25 | 40 | 40 | 40 | 40 | 40 | 40 | 40 |
| | | | Pulp conc. | % | 10 | 10 | 10 | 2 | 35 | 10 | 10 | 10 | 10 | 10 | 10 | 10 | 10 | 10 |
| | | | pH | - | 3.0 | 5.5 | 8.0 | 3.5 | 5.5 | 5.5 | 5.5 | 5.5 | 5.5 | 3.5 | 5.5 | 5.5 | 5.5 | 5.5 |
| | | Brightness | | % | 87.4 | 87.8 | 88.1 | 87.6 | 88.0 | 87.5 | 88.0 | 88.2 | 87.5 | 87.2 | 87.8 | 87.8 | 87.8 | - |
| Pulp sheet for fluff pulp | Basis weight | | | g/m2 | 750 | 750 | 750 | 750 | 750 | 750 | 750 | 750 | 750 | 750 | 750 | 750 | 750 | 750 |
| | Hardwood pulp | | | % | 0 | 0 | 0 | 0 | 0 | 0 | 0 | 0 | 0 | 0 | 5 | 15 | 30 | 15 |
| | Softwood pulp | | | % | 100 | 100 | 100 | 100 | 100 | 100 | 100 | 100 | 100 | 100 | 95 | 85 | 70 | 85 |
| | Extract | pH | | - | 7.2 | 8.3 | 9.1 | 8.6 | 7.9 | 8.7 | 7.8 | 7.7 | 9.0 | 8.5 | 8.2 | 7.6 | 7.4 | 7.6 |
| | Brghtness | | | % | 87.4 | 87.8 | 88.1 | 87.6 | 88.0 | 87.5 | 88.0 | 88.2 | 87.5 | 87.2 | 87.8 | 87.7 | 87.6 | 87.6 |
| | Color fading (105°C) | 0 min (blank) | | % | 87.4 | 87.8 | 88.1 | 87.6 | 88.0 | 87.5 | 88.0 | 88.2 | 87.5 | 87.2 | 87.8 | 87.7 | 87.6 | 87.6 |
| | | 15 min | | % | 86.8 | 87.7 | 87.6 | 87.1 | 87.5 | 87.1 | 87.6 | 87.6 | 87.0 | 86.8 | 87.2 | 87.3 | 87.3 | 87.4 |
| | | 30 min | | % | 86.5 | 87.4 | 87.4 | 87.0 | 87.3 | 96.9 | 87.5 | 87.6 | 86.9 | 86.5 | 86.9 | 87.0 | 87.1 | 87.0 |
| | | 60 min | | % | 86.5 | 87.2 | 87.4 | 86.8 | 87.3 | 86.9 | 87.2 | 87.3 | 86.8 | 86.4 | 86.9 | 86.7 | 86.6 | 86.7 |
| | | 120 min | | % | 86.3 | 86.8 | 87.3 | 86.5 | 87.0 | 86.6 | 86.9 | 87.1 | 86.6 | 86.1 | 86.7 | 86.6 | 86.6 | 86.5 |
| | | Brightness changed | | pt | 1.3 | 1.2 | 0.9 | 1.3 | 1.1 | 1.0 | 1.3 | 1.3 | 1.1 | 1.3 | 1.3 | 1.3 | 1.1 | 1.3 |
| | Defibration properties | | | sec | 17 | 17 | 17 | 17 | 17 | 17 | 17 | 17 | 17 | 15 | 21 | 26 | 30 | 26 |
| Fluff pulp + artificial urine | pH | | | - | 6.8 | 7.3 | 7.6 | 7.4 | 7.1 | 7.4 | 7.0 | 7.0 | 7.6 | 7.5 | 7.4 | 7.0 | 7.0 | 7.0 |
| Fluff pulp + SAP | rewet percentage | | | % | 26 | 23 | 19 | 22 | 24 | 23 | 24 | 25 | 19 | 21 | 19 | 16 | 14 | 16 |
| Susceptibility to skin irritation | | | | - | B | B | A | B | A | B | A | B | A | B | A | A | A | A |

**[Table 2]**

| | | | | | Comp.Ex. 1 | Comp.Ex. 2 | Comp.Ex. 3 | Comp.Ex. 4 | Comp.Ex. 5 |
|---|---|---|---|---|---|---|---|---|---|
| Hardwood pulp | Tree type | genus Acacia | | % | - | - | - | 70 | 70 |
| | | genus Eucalyptus | | % | - | - | - | 30 | 30 |
| | Heat treatment | Cond. | Time | h | - | - | - | 12 | 12 |
| | | | Temperature | °C | - | - | - | 40 | 40 |
| | | | Pulp conc. | % | - | - | - | 10 | 10 |
| | | | pH | - | - | - | - | 5.5 | 5.5 |
| | | Brightness | | % | - | - | - | 87.4 | 87.4 |
| Softwood pulp | Tree type | genus Pseudotsuga | | % | 70 | 70 | 70 | 70 | - |
| | | genus Pinus | | % | 30 | 30 | 30 | 30 | - |
| | Heat treatment | Cond. | Time | h | - | 12 | 12 | 12 | - |
| | | | Temperature | °C | - | 40 | 40 | 40 | - |
| | | | Pulp conc. | % | - | 10 | 10 | 10 | - |
| | | | pH | - | - | 1.0 | 10 | 5.5 | - |
| | | Brightness | | % | - | 86.9 | 87.3 | 87.8 | - |
| Pulp sheet for fluff pulp | Basis weight | | | g/m2 | 750 | 750 | 750 | 750 | 750 |
| | Hardwood pulp | | | % | 0 | 0 | 0 | 50 | 100 |
| | Softwood pulp | | | % | 100 | 100 | 100 | 50 | 0 |
| | Extract | pH | | - | 6.3 | 5.9 | 10.3 | 6.8 | 6.5 |
| | Brghtness | | | % | 88.2 | 86.9 | 87.3 | 87.6 | 87.4 |
| | Color fading (105°C) | 0 min (blank) | | % | 88.2 | 86.9 | 87.3 | 87.6 | 87.4 |
| | | 15 min | | % | 86.8 | 86.7 | 87.0 | 87.1 | 87.0 |
| | | 30 min | | % | 86.6 | 86.2 | 86.8 | 87.0 | 86.9 |
| | | 60 min | | % | 86.3 | 86.0 | 86,2 | 86.7 | 86.6 |
| | | 120 min | | % | 86.0 | 85.7 | 86.0 | 86.5 | 86.3 |
| | | Brightness changed | | pt | 2.5 | 1.4 | 1.5 | 1.3 | 1.3 |
| | Defibration properties | | | sec | 17 | 17 | 17 | 49 | 80 |
| Fluff pulp + artificial urine | pH | | | - | 6.5 | 6.2 | 8.4 | 6.4 | 6.4 |
| Fluff pulp + SAP | rewet percentage | | | % | 31 | 34 | 17 | 7 | 4 |
| Susceptibility to skin irritation | | | | - | C | D | C | C | C |

Compared to the comparative examples, in the examples, there were obtained pulp sheets for fluff pulp that suppress fading and have excellent defibration properties. Moreover, in the examples, there were obtained fluff mats that can suppress the occurrence of skin irritation.

### (Second embodiment)

### [Preparation of softwood pulp]

### [Cooking]

After the chips of genus Pseudotsuga had been mixed with the chips of genus Pinus at an oven-dry mass ratio of 60 : 40, 200 g (oven-dry mass) of the mixed chips were collected and placed in a 2.5-L autoclave, and kraft cooking was then carried out under the conditions of: a liquid ratio of 5, a degree of sulfidation of a cooking liquid of 28%, an alkali addition percentage of 22%, a cooking temperature of 165°C, and a cooking time of 120 minutes. Thereafter, a black liquor and a pulp were separated from each other, and the pulp was refined using a flat screen equipped with a 10-cut screen plate to obtain an unbleached pulp with a kappa number of about 30.

### [Oxygen delignification]

The unbleached pulp (70.0 g) was collected at an oven-dry mass, and caustic soda was then added to the unbleached pulp, so that the amount of the caustic soda became 2.0% by mass with respect to the mass of the oven-dried pulp. Subsequently, the obtained mixture was diluted with ion exchange water to adjust the pulp concentration to 10% by mass. This reaction mixture was placed in a heat-type autoclave, and 99.9% commercially available compressed oxygen gas was then injected into the autoclave to set the gauge pressure at 0.5 MPa. Thereafter, oxygen delignification was carried out at 95°C for 60 minutes. The obtained pulp was diluted with ion exchange water to 3% by mass, and was then dehydrated and washed in a Buchner funnel.

### [Bleaching]

The kraft pulp (60 g) after alkaline oxygen delignification was collected at an oven-dry mass, and was then placed in a plastic bag. The pulp concentration was adjusted to 10% by mass using ion exchange water, and chlorine dioxide was added to the pulp so that the amount of chlorine dioxide became 2.0% by mass with respect to the mass of the oven-dried pulp. This reaction mixture was immersed in a thermostatic water bath at 70°C for 60 minutes to carry out a D0 stage treatment. Thereafter, the obtained pulp was diluted with ion exchange water to 3% by mass, and was then dehydrated and washed in a Buchner funnel.

The pulp after the D0 stage was placed in a plastic bag, and the pulp concentration was adjusted to 10% by mass by addition of ion exchange water. Thereafter, caustic soda and hydrogen peroxide were added to the pulp, so that the amount of the caustic soda became 1.0% by mass and the amount of the hydrogen peroxide became 0.2% by mass with respect to the mass of the oven-dried pulp, followed by full mixing. Thereafter, the reaction mixture was immersed in a thermostatic water bath at 70°C for 90 minutes to carry out an E/P stage treatment. The obtained pulp was diluted with ion exchange water to 3% by mass, and was then dehydrated and washed in a Buchner funnel.

The pulp after the E/P stage was placed in a plastic bag, and the pulp concentration was adjusted to 10% by mass by addition of ion exchange water. Thereafter, caustic soda and hydrogen peroxide were added to the pulp, so that the amount of the caustic soda became 1.0% by mass and the amount of the hydrogen peroxide became 0.3% by mass with respect to the mass of the oven-dried pulp, followed by fully mixing. Thereafter, the reaction mixture was immersed in a thermostatic water bath at 70°C for 90 minutes to carry out a P0 stage treatment. The obtained pulp was diluted with ion exchange water to 3% by mass, and was then dehydrated and washed in a Buchner funnel.

The pulp after the P0 stage was placed in a plastic bag, and the pulp concentration was adjusted to 10% by mass by addition of ion exchange water. Thereafter, chlorine dioxide was added to the pulp, so that the amount of the chlorine dioxide became 0.2% by mass with respect to the mass of the oven-dried pulp, and the reaction mixture was then immersed in a thermostatic water bath at 70°C for 60 minutes to carry out a D1 stage bleaching. The obtained bleached softwood pulp was diluted with ion exchange water to 3% by mass, and was then dehydrated and washed in a Buchner funnel.

### [Color tone adjustment step]

The bleached softwood pulp was placed in a beaker, and the pulp concentration thereof was then adjusted to 10% by mass using ion exchange water. Thereafter, the slurry was placed in a shaker heated to 60°C, and hydrogen peroxide was then added thereto to a concentration of 1.0% relative to the mass of the oven-dried pulp. Thereafter, the beaker was covered with a lid and was then subjected to a shaking treatment for 60 minutes to adjust the color tone. The resulting color tone-adjusted pulp was diluted to 3% by mass with ion exchange water, and was then dehydrated and washed in a Buchner funnel.

### [Preparation of hardwood pulp]

### [Cooking]

After the chips of genus Acacia had been mixed with the chips of genus Eucalyptus at an oven-dry mass ratio of 70 : 30, 200 g (oven-dry mass) of the mixed chips were collected and placed in a 2.5-L autoclave, and kraft cooking was then carried out under the conditions of: a liquid ratio of 5, a degree of sulfidation of a cooking liquid of 28%, an alkali addition percentage of 20%, a cooking temperature of 155°C, and a cooking time of 120 minutes. Thereafter, a black liquor and a pulp were separated from each other, and the pulp was refined using a flat screen equipped with a 8-cut screen plate to obtain an unbleached pulp with a kappa number of about 18.

### [Oxygen delignification]

The unbleached pulp (70.0 g) was collected at an oven-dry mass, and caustic soda was then added to the unbleached pulp, so that the amount of the caustic soda became 2.0% by mass with respect to the mass of the oven-dried pulp. Subsequently, the obtained mixture was diluted with ion exchange water to adjust the pulp concentration to 10% by mass. This reaction mixture was placed in a heat-type autoclave, and 99.9% commercially available compressed oxygen gas was then injected into the autoclave to set the gauge pressure at 0.5 MPa. Thereafter, oxygen delignification was carried out at 95°C for 60 minutes. The obtained pulp was diluted with ion exchange water to 3% by mass, and was then dehydrated and washed in a Buchner funnel.

### [Bleaching]

The kraft pulp (60 g) after alkaline oxygen delignification was collected at an oven-dry mass, and was then placed in a plastic bag. The pulp concentration was adjusted to 10% by mass using ion exchange water, and chlorine dioxide was added to the pulp so that the amount of chlorine dioxide became 1.0% by mass with respect to the mass of the oven-dried pulp. This reaction mixture was immersed in a thermostatic water bath at 70°C for 60 minutes to carry out a D0 stage treatment. Thereafter, the obtained pulp was diluted with ion exchange water to 3% by mass, and was then dehydrated and washed in a Buchner funnel.

The pulp after the D0 stage was placed in a plastic bag, and the pulp concentration was adjusted to 10% by mass by addition of ion exchange water. Thereafter, caustic soda and hydrogen peroxide were added to the pulp, so that the amount of the caustic soda became 1.0% by mass and the amount of the hydrogen peroxide became 0.2% by mass with respect to the mass of the oven-dried pulp, followed by full mixing. Thereafter, the reaction mixture was immersed in a thermostatic water bath at 70°C for 90 minutes to carry out an E/P stage treatment. The obtained pulp was diluted with ion exchange water to 3% by mass, and was then dehydrated and washed in a Buchner funnel.

The pulp after the E/P stage was placed in a plastic bag, and the pulp concentration was adjusted to 10% by mass by addition of ion exchange water. Thereafter, chlorine dioxide was added to the pulp, so that the amount of the chlorine dioxide became 0.2% by mass with respect to the mass of the oven-dried pulp, and the reaction mixture was then immersed in a thermostatic water bath at 70°C for 60 minutes to carry out a D1 stage bleaching. The obtained bleached hardwood pulp was diluted with ion exchange water to 3% by mass, and was then dehydrated and washed in a Buchner funnel.

### [Color tone adjustment step]

The bleached hardwood pulp was placed in a beaker, and the pulp concentration thereof was then adjusted to 10% by mass using ion exchange water. Thereafter, the slurry was placed in a shaker heated to 60°C, and hydrogen peroxide was then added thereto to a concentration of 1.0% relative to the mass of the oven-dried pulp. Thereafter, the beaker was covered with a lid and was then subjected to a shaking treatment for 60 minutes to adjust the color tone. The resulting color tone-adjusted pulp was diluted to 3% by mass with ion exchange water, and was then dehydrated and washed in a Buchner funnel.

### (Example 101)

After the color tone adjustment step, the bleached softwood pulp was mixed with the bleached hardwood pulp at an oven-dry mass ratio of 100 : 0, so as to obtain a slurry having a pulp concentration of 1% by mass. Thereafter, using a standard circular hand papermaking machine (manufactured by KUMAGAI RIKI KOGYO Co., Ltd.), the slurry was subjected to papermaking, so that the basis weight became 750 g/m², thereby obtaining a pulp sheet for fluff pulp.

### (Example 102)

A pulp sheet for fluff pulp was obtained by performing the same operations as those in Example 101, with the exception that the hydrogen peroxide added in the color tone adjustment step after obtaining the bleached softwood pulp was set to be 0.5%.

### (Example 103)

A pulp sheet for fluff pulp was obtained by performing the same operations as those in Example 101, with the exception that the hydrogen peroxide added in the color tone adjustment step after obtaining the bleached softwood pulp was set to be 1.5%.

### (Example 104)

A pulp sheet for fluff pulp was obtained by performing the same operations as those in Example 101, with the exception that the pulp concentration in the color tone adjustment step after obtaining the bleached softwood pulp was set to be 1% by mass.

### (Example 105)

A pulp sheet for fluff pulp was obtained by performing the same operations as those in Example 101, with the exception that the pulp concentration in the color tone adjustment step after obtaining the bleached softwood pulp was set to be 20% by mass.

### (Example 106)

A pulp sheet for fluff pulp was obtained by performing the same operations as those in Example 101, with the exception that the shaking treatment time in the color tone adjustment step after obtaining the bleached softwood pulp was set to be 90 minutes.

### (Example 107)

A pulp sheet for fluff pulp was obtained by performing the same operations as those in Example 101, with the exception that the shaking treatment time in the color tone adjustment step after obtaining the bleached softwood pulp was set to be 120 minutes.

### (Example 108)

A pulp sheet for fluff pulp was obtained by performing the same operations as those in Example 101, with the exception that the temperature in the color tone adjustment step after obtaining the bleached softwood pulp was set to be 80°C.

### (Example 109)

A pulp sheet for fluff pulp was obtained by performing the same operations as those in Example 101, with the exception that the bleached softwood pulp was produced by mixing the chips of genus Pinus with the chips of genus Pseudotsuga at an oven-dry mass ratio of 70 : 30.

### (Example 110)

A pulp sheet for fluff pulp was obtained by performing the same operations as those in Example 101, with the exception that the bleached softwood pulp after the color tone adjustment step was mixed with the bleached hardwood pulp after the color tone adjustment step at an oven-dry mass ratio of 95 : 5 during papermaking.

### (Example 111)

A pulp sheet for fluff pulp was obtained by performing the same operations as those in Example 101, with the exception that the bleached softwood pulp after the color tone adjustment step was mixed with the bleached hardwood pulp after the color tone adjustment step at an oven-dry mass ratio of 85 : 15 during papermaking.

### (Example 112)

A pulp sheet for fluff pulp was obtained by performing the same operations as those in Example 101, with the exception that the bleached softwood pulp after the color tone adjustment step was mixed with the bleached hardwood pulp after the color tone adjustment step at an oven-dry mass ratio of 70 : 30 during papermaking.

### (Example 113)

A pulp sheet for fluff pulp was obtained by performing the same operations as those in Example 101, with the exceptions that the bleached softwood pulp was mixed with the bleached hardwood pulp at an oven-dry mass ratio of 85 : 15 before the color tone adjustment step, that the color tone adjustment step was carried out at the same ratio as described above, and that papermaking was then carried out.

### (Comparative Example 101)

A pulp sheet for fluff pulp was obtained by performing the same operations as those in Example 101, with the exception that 1.0% chlorine dioxide was added instead of hydrogen peroxide in the color tone adjustment step after obtaining the bleached softwood pulp.

### (Comparative Example 102)

A pulp sheet for fluff pulp was obtained by performing the same operations as those in Example 101, with the exception that the bleached softwood pulp was mixed with the bleached hardwood pulp at an oven-dry mass ratio of 50 : 50 during papermaking.

### (Comparative Example 103)

A pulp sheet for fluff pulp was obtained by performing the same operations as those in Example 101, with the exception that the bleached softwood pulp was mixed with the bleached hardwood pulp at an oven-dry mass ratio of 0 : 100 during papermaking.

### (Measurement/Evaluation)

### [ISO brightness]

The ISO brightness of the pulp sheet for fluff pulp was measured in accordance with JIS P 8148: 2018. At that time, the ISO brightness of the pulp sheet for fluff pulp was measured before and after being heated at 105°C for 120 minutes. After that, the amount of ISO brightness changed was calculated according to the following equation:$Amount of ISO brightness changed = \left(B0\left(%\right)-B1\left(%\right)\right) / B 0 \left(%\right) \times 100 .$

In the above equation, B1 represents the ISO brightness of the pulp sheet for fluff pulp measured in accordance with JIS P 8154-1: 2008, after being heated at 105°C for 2 hours, and B0 represents the ISO brightness of the pulp sheet for fluff pulp before the heat treatment.

### [Defibration properties]

The defibration properties of the pulp sheet for fluff pulp were evaluated under an environment of 23°C and a relative humidity of 50%. The pulp sheet for fluff pulp, which had been humidity-conditioned under an environment of 23°C and a relative humidity of 50%, was cut into a 10 mm x 10 mm square, and 3 g thereof was weighed out and was put into a Wonder Blender (manufactured by OSAKA CHEMICAL Co., Ltd., model: WB-1, rotation number: 25000 rpm) while avoiding any bias. The pulp was defibrated for a predetermined time in 5-second intervals to obtain a cotton-like pulp (hereinafter, the pulp made into a cotton-like form by defibration is called "fluff pulp"). Subsequently, the fluff pulp was immersed in water and was gently stirred with a stick, and then, using a standard circular hand papermaking machine, a handmade paper with a basis weight of 25 g/m² for use in evaluation of defibration properties was prepared. The handmade paper for use in evaluation of defibration properties was visually observed, and the defibration time (sec) required for the undefibrated material to disappear was used as an indicator for evaluation of the defibration properties.

**[Table 3]**

| | | | | | Example 101 | Example 102 | Example 103 | Example 104 | Example 105 | Example 106 | Example 107 | Example 108 | Example 109 | Example 110 | Example 111 | Example 112 | Example 113 |
|---|---|---|---|---|---|---|---|---|---|---|---|---|---|---|---|---|---|
| Hardwood pulp | Tree type | genus Acacia | | % | - | - | - | - | - | - | - | - | - | 70 | 70 | 70 | 70 |
| | | genus Eucalyptus | | % | - | - | - | - | - | - | - | - | - | 30 | 30 | 30 | 30 |
| | Color tone adjustiment step | Drug addition pecantage | ClO₂ | % | - | - | - | - | - | - | - | - | - | 0 | 0 | 0 | 0 |
| | | | H₂O₂ | % | - | - | - | - | - | - | - | - | - | 1.0 | 1.0 | 1.0 | 1.0 |
| | | | NaOH | % | - | - | - | - | - | - | - | - | - | 0.5 | 0.5 | 0.5 | 0.5 |
| | | Pulp conc. | | % | - | - | - | - | - | - | - | - | - | 10 | 10 | 10 | 10 |
| | | Time | | min | - | - | - | - | - | - | - | - | - | 60 | 60 | 60 | 60 |
| | | Temperature | | °C | - | - | - | - | - | - | - | - | - | 60 | 60 | 60 | 60 |
| | | Final pH | | - | - | - | - | - | - | - | - | - | - | 11.2 | 11.2 | 11.2 | - |
| | | Brightness | | % | - | - | - | - | - | - | - | - | - | 87.6 | 87.6 | 87.6 | - |
| Softwood pulp | Tree type | genus Pseudotsuga | | % | 60 | 60 | 60 | 60 | 60 | 60 | 60 | 60 | 30 | 60 | 60 | 60 | 60 |
| | | genus Pinus | | % | 40 | 40 | 40 | 40 | 40 | 40 | 40 | 40 | 70 | 40 | 40 | 40 | 40 |
| | Color tone adjustment step | Drug addition percentage | ClO₂ | % | 0 | 0 | 0 | 0 | 0 | 0 | 0 | 0 | 0 | 0 | 0 | 0 | 0 |
| | | | H₂O₂ | % | 1.0 | 0.5 | 1.5 | 1.0 | 1.0 | 1.0 | 1.0 | 1.0 | 1.0 | 1.0 | 1.0 | 1.0 | 1.0 |
| | | | NaOH | % | 0.5 | 0.3 | 0.7 | 0.5 | 0.5 | 0.5 | 0.5 | 0.5 | 0.5 | 0.5 | 0.5 | 0.5 | 0.5 |
| | | Pulp conc. | | % | 10 | 10 | 10 | 1 | 20 | 10 | 10 | 10 | 10 | 10 | 10 | 10 | 10 |
| | | Time | | min | 60 | 60 | 60 | 60 | 60 | 90 | 120 | 60 | 60 | 60 | 60 | 60 | 60 |
| | | Temperature | | °C | 60 | 60 | 60 | 60 | 60 | 60 | 60 | 80 | 60 | 60 | 60 | 60 | 60 |
| | | Final pH | | - | 11.4 | 10.6 | 12.4 | 11.5 | 11.3 | 11.3 | 11.1 | 11.1 | 11.4 | 11.4 | 11.4 | 11.4 | - |
| | | Brightness | | % | 88.0 | 86.5 | 88.4 | 88.2 | 87.5 | 87.6 | 87.3 | 88.5 | 87.6 | 88.0 | 88.0 | 88.0 | - |
| Pulp sheet for fluff pulp | Basis weight | | | g/m2 | 750 | 750 | 750 | 750 | 750 | 750 | 750 | 750 | 750 | 750 | 750 | 750 | 750 |
| | Hardwood pulp | | | % | 0 | 0 | 0 | 0 | 0 | 0 | 0 | 0 | 0 | 5 | 15 | 30 | 15 |
| | Softwood pulp | | | % | 100 | 100 | 100 | 100 | 100 | 100 | 100 | 100 | 100 | 95 | 85 | 70 | 85 |
| | Color fading (105°C) | 0 min (blank) | | % | 88.0 | 86.5 | 88.4 | 88.2 | 87.5 | 87.6 | 87.3 | 88.5 | 87.7 | 88.0 | 87.9 | 87.8 | 87.9 |
| | | 120 min | | % | 87.2 | 85.6 | 87.3 | 87.4 | 86.6 | 86.9 | 86.4 | 88.0 | 86.8 | 87.0 | 86.9 | 86.8 | 86.9 |
| | | Brightness changed | | % | 0.9 | 1.1 | 1.3 | 1.0 | 1.0 | 0.8 | 1.1 | 0.6 | 1.0 | 1.0 | 1.1 | 1.1 | 1.1 |
| | Defibration properties | | | sec | 17 | 17 | 17 | 17 | 17 | 17 | 17 | 17 | 17 | 21 | 26 | 33 | 26 |

**[Table 4]**

| | | | | | Comp.Ex. 101 | Comp.Ex. 102 | Comp.Ex. 103 |
|---|---|---|---|---|---|---|---|
| Hardwood pulp | Tree type | genus Acacia | | % | - | 70 | 70 |
| | | genus Eucalyptus | | % | - | 30 | 30 |
| | Color tone adjustment step | Drug addition percentage | ClO₂ | % | - | - | - |
| | | | H₂O₂ | % | - | 1.0 | 1.0 |
| | | | NaOH | % | - | 0.5 | 0.5 |
| | | Pulp conc. | | % | - | 10 | 10 |
| | | Time | | min | - | 60 | 60 |
| | | Temperature | | °C | - | 60 | 60 |
| | | Final pH | | - | - | 11.2 | 11.2 |
| | | Brightness | | % | - | 87.6 | 87.6 |
| Softwood pulp | Tree type | genus Pseudotsuga | | % | 60 | 60 | - |
| | | genus Pinus | | % | 40 | 40 | - |
| | Color tone adjustment step | Drug addition percentage | ClO₂ | % | 1.0 | 0 | - |
| | | | H₂O₂ | % | 0.0 | 1.0 | - |
| | | | NaOH | % | 0.5 | 0.5 | - |
| | | Pulp conc. | | % | 10 | 10 | - |
| | | Time | | min | 60 | 60 | - |
| | | Temperature | | °C | 60 | 60 | - |
| | | Final pH | | - | 4.8 | 11.4 | - |
| | | Brightness | | % | 88.7 | 88.0 | - |
| Pulp sheet for fluff pulp | Basis weight | | | g/m2 | 750 | 750 | 750 |
| | Hardwood pulp | | | % | 0 | 50 | 100 |
| | Softwood pulp | | | % | 100 | 50 | 0 |
| | Color fading (105°C) | 0 min (blank) | | % | 88.7 | 87.8 | 87.6 |
| | | 120 min | | % | 86.5 | 86.8 | 86.6 |
| | | Brightness changed | | % | 2.5 | 1.1 | 1.2 |
| | Defibration properties | | | sec | 17 | 49 | 80 |

Compared to the comparative examples, in the examples, pulp sheets for fluff pulp that suppressed fading and had excellent defibration properties could be obtained.

### Reference Signs List

- 5: Pulp defibrating machine
- 10: Polypropylene powder funnel
- 20: Column
- 22, 24: Stainless steel sieve
- 30: Polypropylene powder funnel
- 40: Cleaner (suction machine)
- 22: Stainless steel sieve with sieve opening of 180 µm
- 100: Fluff mat-producing device

## Claims

1. A pulp sheet for fluff pulp, in which the pH of a hot water extract thereof, measured in accordance with JIS P 8133-2: 2013, is pH 7.0 to 9.5.

2. The pulp sheet for fluff pulp according to claim 1, wherein the ISO brightness measured in accordance with JIS P 8148: 2018 is 80% or more.

3. The pulp sheet for fluff pulp according to claim 1, wherein
the pulp sheet for fluff pulp comprises a softwood pulp, and
the content of a hardwood pulp with respect to the total pulp components in the pulp sheet for fluff pulp is 30% by mass or less.

4. The pulp sheet for fluff pulp according to claim 1, wherein
the pulp sheet for fluff pulp comprises a softwood pulp, and
the content of a hardwood pulp with respect to the total pulp components in the pulp sheet for fluff pulp is 15% by mass or less.

5. A pulp sheet for fluff pulp, comprising a softwood pulp, in which
the content of a hardwood pulp with respect to the total pulp components in the pulp sheet for fluff pulp is 30% by mass or less, and
the amount of ISO brightness changed, calculated according to the following equation, is 1.5% or less: $Amount \left(%\right) of ISO brightness changed = \left(B0 \left(%\right)-B1 \left(%\right)\right) / B 0 \left(%\right) \times 100 ,$
wherein B1 represents the ISO brightness of the pulp sheet for fluff pulp measured in accordance with JIS P 8154-1: 2008, after being heated at 105°C for 2 hours, and B0 represents the ISO brightness of the pulp sheet for fluff pulp before the heat treatment.

6. The pulp sheet for fluff pulp according to claim 5, wherein
the pulp sheet for fluff pulp comprises a softwood pulp, and
the content of a hardwood pulp with respect to the total pulp components in the pulp sheet for fluff pulp is 15% by mass or less.

7. A fluff mat formed from the pulp sheet for fluff pulp according to any one of claims 1 to 6.

8. The fluff mat according to claim 7, wherein after 5 parts by mass of the fluff mat is impregnated with 20 parts by mass of artificial urine containing 1.94% by mass of urea, 0.8040% by mass of sodium chloride, 0.2045% by mass of magnesium sulfate heptahydrate, and 0.0835% by mass of calcium chloride dihydrate, and the resulting fluff mat is then left at rest at 40°C for 1 hour in a sealed state, the pH of a liquid squeezed from the fluff mat is pH 6.6 or more.

9. An absorbent article, comprising the fluff mat according to claim 7.

10. A method for producing a pulp sheet for fluff pulp, comprising
cooking a pulp raw material to obtain an unbleached pulp,
bleaching,
heating, and forming a sheet formation, wherein
the heating is provided after the bleaching.

11. The method for producing a pulp sheet for fluff pulp according to claim 10, wherein, in the heating, the heat treatment is carried out, so that the pulp slurry temperature becomes 30°C or higher and 100°C or lower.

12. The method for producing a pulp sheet for fluff pulp according to claim 10, wherein the heat treatment time in the heating is 10 minutes or more and 120 minutes or less.

13. The method for producing a pulp sheet for fluff pulp according to claim 10, wherein the concentration of the pulp slurry in the heating is 0.5% by mass or more and 40% by mass or less.

14. The method for producing a pulp sheet for fluff pulp according to claim 10, wherein the pH of the pulp slurry in the heating is pH 2.0 or more and pH 9.0 or less.
